# EUROPÄISCHE PATENTANMELDUNG

(11) **EP 1 222 904 A1**
(43) Veröffentlichungstag der Anmeldung: **17.07.2002**
(21) Anmeldenummer: 01890341.9
(22) Anmeldetag: 18.12.2001
(51) Int. Cl.: A61F 5/56

(54) **Vorrichtung zur Schnarchverhinderung**

(30) Priorität: 19.12.2000 AT 21092000
(71) Anmelder: Pieringer, Josef, 4850 Timelkam (AT); Brandstätter, Thomas, 4840 Lenzing (AT); Matejo, Franz, 4880 St. Georgen im Attergau (AT)
(72) Erfinder: Pieringer, Josef, 4850 Timelkam (AT); Brandstätter, Thomas, 4840 Lenzing (AT); Matejo, Franz, 4880 St. Georgen im Attergau (AT)
(74) Vertreter: Hübscher, Helmut, Dipl.-Ing.

(57) **Zusammenfassung**

Es wird eine Vorrichtung zur Schnarchverhinderung mit einem über eine Steuereinrichtung (4) ansteuerbaren Vibrator zur Schwingungsübertragung auf einen Schlafenden beschrieben. Um vorteilhafte Konstruktionsbedingungen zu schaffen, wird vorgeschlagen, daß der Vibrator aus einem mit einer Schwingungsübertragungsplatte (1) verbundenen, elektrischen Unwuchtmotor (3) besteht und daß die Schwingungsübertragungsplatte (1) eine Unterlage für einen Kopfpolster (11) bildet.

## Beschreibung

Die Erfindung bezieht sich auf eine Vorrichtung zur Schnarchverhinderung mit einem über eine Steuereinrichtung ansteuerbaren Stelleinrichtung für eine Kopfauflage eines Schlafenden.

Um einen Schlafenden am Schnarchen zu hindern, ist es bekannt, in Abhängigkeit von einer Schnarchgeräuscherfassung dem Schlafenden ein Signal zukommen zu lassen, das ihn veranlaßt, das erschlaffte Gaumensegel anzuspannen und mit dem Schnarchen aufzuhören, ohne aufzuwachen. Zu diesem Zweck wurde bereits vorgeschlagen (DE 196 26 273 A1), den Schlafenden über einen Kopfhörer oder einen Lautsprecher zu beschallen, was jedoch zu einer Belästigung anderer Personen führen kann, selbst wenn der Lautsprecher zusammen mit einem Mikrofon und einer Auswerteschaltung zur Ansteuerung des Lautsprechers in einem Kopfpolster untergebracht wird. Eine weitere Möglichkeit der Beeinflussung eines Schlafenden beim Einsetzen eines Schnarchens besteht darin, über einen Vibrator auf den Körper Schwingungen zu übertragen (DE 200 05 911 U1). Nachteilig bei solchen Vibratoren ist allerdings, daß sie wie Kopfhörer am Körper getragen werden müssen, was häufig als störend empfunden wird, insbesondere wenn im Sinne einer einfachen Handhabung und eines geringen Konstruktionsaufwandes der Vibrator mit dem ihn steuernden Mikrofon zu einer Baueinheit zusammengefaßt wird. Dies gilt auch für die Anordnung einer solchen Baueinheit in einem Armband (DE 20001 557 U1), das den zusätzlichen Nachteil aufweist, daß sich die Lage des Armes gegenüber dem Kopf ändert, was zu Schwierigkeiten bei der Einstellung der Empfindlichkeit des Mikrofones und damit zur Unterscheidung von Schnarchgeräuschen gegenüber anderen Geräuschen über den Schallpegel führt.

Darüberhinaus ist es bekannt (GB 2 208 003 A, US 4 941 478 A), durch eine Verlagerung des Kopfes und der damit verbundenen Verlagerung des Zungenhintergrundes und des erschlafften Gaumensegels ein Schnarchen zu unterbrechen. Zu diesem Zweck wird eine Kopfauflage über einen Motor um wenigstens eine Achse verschwenkt, wenn ein Schnarchgeräusch über ein Mikrofon empfangen wird. Nachteilig bei diesen bekannten Vorrichtungen ist vor allem, daß die natürliche Kopfhaltung verändert und die gewohnte Liegestellung des Schlafenden gestört wird. Durch die zwangsweise Veränderung der Kopflage besteht die Gefahr, daß der Schlafende aufgeweckt wird, was zu vermeiden ist. Außerdem kann es zu Muskelverspannungen im Nackenbereich aufgrund der aufgezwungenen Kopfhaltung kommen.

Der Erfindung liegt somit die Aufgabe zugrunde, eine Vorrichtung zur Schnarchverhinderung der eingangs geschilderten Art so auszugestalten, daß auf den Schlafenden in einer ihn nicht beeinträchtigenden Weise Einfluß zur Schnarchunterdrückung genommen werden kann.

Die Erfindung löst die gestellte Aufgabe dadurch, daß die Stelleinrichtung die Kopfauflage nach einer Schwingungs- oder Impulsanregung in die Ausgangslage zurückstellt.

Da der Kopf des Schlafenden über die Kopfauflage mit Schwingungen oder Impulsen beaufschlagt wird, deren Amplitude so ausgewählt wird, daß sie den Schlaf nicht unterbrechen, aber eine ausreichende Größe zum Anspannen des erschlafften Gaumensegels aufweisen, wird der Schnarchvorgang durch eine solche Schwingungs- oder Impulsanregung der Kopfauflage wirksam unterbrochen. Obwohl sich der Schlafende frei und ungehindert bewegen kann, ist für die zur Unterdrückung eines Schnarchens erforderliche Schwingungs- bzw. Impulsübertragung im Bereich der gesamten Kopfauflage gesorgt. Die Stelleinrichtung führt die Kopfauflage nach der Schwingungs- bzw. Impulsanregung wieder in die Ausgangsstellung zurück, so daß mit der Schwingungs- oder Impulsübertragung auf den Kopf des Schlafenden keine die gewählte Schlafhaltung beeinträchtigende Kopfverlagerung verbunden ist.

Zur Schwingungsübertragung kann ein mit einer Schwingungsübertragungsplatte verbundener, elektrischer Unwuchtmotor vorgesehen werden, wobei die Schwingungsübertragungsplatte eine Unterlage für einen Kopfpolster bildet. Die Gewichtsbelastung des Polsters durch den Kopf des Schlafenden bedingt im jeweiligen Auflagebereich des Kopfes eine Verdichtung des Kopfpolsters mit der Wirkung einer besseren Schwingungsübertragung von der Schwingungsübertragungsplatte über den Kopfpolster auf den Kopf des Schlafenden.

Um die Kopfauflage nicht durch gegen den Kopfpolster vorstehende Konstruktionsteile zu beeinträchtigen, kann der Unwuchtmotor in einer Ausnehmung der Schwingungsübertragungsplatte vorgesehen sein, die somit eine im wesentlichen ebene Auflagefläche für den Kopfpolster bilden kann. Dies gilt insbesondere dann, wenn der Unwuchtmotor mit der Steuereinrichtung in einem Gehäuse angeordnet wird, das in die Ausnehmung der Schwingungsübertragungsplatte formschlüssig eingreift, weil in diesem Fall das Gehäuse die Ausnehmung der Schwingungsübertragungsplatte bündig abschließt. Außerdem bringt der Formschluß zwischen dem Gehäuse und der Schwingungsübertragungsplatte günstige Bedingungen für die Schwingungsübertragung auf die Schwingungsübertragungsplatte mit sich.

Ist die Drehzahl des Unwuchtmotors über einen Wahlschalter einstellbar, so können die Schwingungsamplituden an die jeweiligen Verhältnisse angepaßt werden. Mit dieser Einstellmöglichkeit der Schwingungsamplituden kann somit einerseits auf die Empfindlichkeit des Schlafenden gegenüber solchen Schwingungen und anderseits auf die Schwingungsdämpfung durch den Kopfpolster vorteilhaft eingegangen werden.

Um eine wirksame Schnarchverhinderung zu erreichen, ist eine Schnarcherfassung nicht zwingend erforderlich. Der Unwuchtmotor könnte ja in sich periodisch wiederholenden Intervallen ein- und ausgeschaltet werden. Um die Schwingungsbelastung des Schlafenden gering zu halten, empfiehlt sich allerdings eine an sich bekannte Ansteuerung des Unwuchtmotors über eine Erfassung der Schnarchgeräusche durch ein Mikrofon. Obwohl die Schwingungsübertragungsplatte eine Unterlage für den Kopfpolster bildet und daher eine örtliche Zuordnung zischen dem Kopf und der Schwingungsübertragungsplatte vorgegeben ist, ist hinsichtlich der Erfassung des Schnarchgeräusches die jeweilige Drehlage des Kopfes zu berücksichtigen. Aus diesem Grunde werden vorzugsweise zwei Mikrofone in je einer Aussparung der Schwingungsübertragungsplatte im Bereich von einander gegenüberliegenden Seitenrändern vorgesehen, so daß über eines der beiden Mikrofone die Schnarchgeräusche bei einer vergleichsweise niedrigen Empfindlichkeit der Mikrofone erfaßt werden können, um über den Schallpegel eine einfache Unterscheidung von anderen äußeren Geräuschen zu erhalten, die aufgrund der geringen Empfindlichkeit der Mikrofone nicht zu einem Auslösesignal für den Unwuchtmotor führen.

Besonders einfache Handhabungsverhältnisse ergeben sich, wenn die Schwingungsübertragungsplatte nicht eine vom Kopfpolster getrennte Unterlage darstellt, sondern mit dem eine nachgiebige Kopfauflage beispielsweise aus Naturlatex bildenden Kopfpolster zu einer Handhabungseinheit verbunden ist. In einem solchen Fall kann eine sonst mögliche Verlagerung des Kopfpolsters gegenüber der Schwingungsübertragungsplatte einfach ausgeschlossen werden.

Eine andere Möglichkeit, Schwingungen bzw. Impulse über eine Kopfauflage auf den Kopf eines Schlafenden zu übertragen, ergibt sich dadurch, daß die Stelleinrichtung aus einer über die Steuereinrichtung an ein aufblasbares Kissen anschließbaren Druckmittelquelle besteht. Durch ein rasches Aufblasen des Kissens und ein langsames Ablassen des Druckmittels kann auf den Schlafenden ein für die Schnarchunterdrückung vorteilhafter Impuls oder eine Impulsfolge übertragen werden, und zwar in der jeweiligen Schlaflage. Eine ähnliche Wirkung kann dadurch erzielt werden, daß zur Impulsübertragung Druckmittel rasch aus dem Kissen abgelassen wird, das anschließend langsam wieder durch Zuführen von Druckmittel aufgeblasen wird, bis der ursprüngliche Ausgangszustand wiederhergestellt ist. Selbstverständlich ist auch eine Kombination dieser Füll- und Ablaßvorgänge möglich. Durch eine gummielastische Ausbildung des Kissens können die beim Aufblasen auftretenden Vorspannungen des Kissens zum rascheren Ablassen des Druckmittels ausgenützt werden.

In der Zeichnung ist der Erfindungsgegenstand beispielsweise dargestellt.

Es zeigen
Fig. 1 eine erfindungsgemäße Vorrichtung in einer schematischen, zum Teil aufgerissenen, blockschaltbildartigen Draufsicht,
Fig. 2 einen Schnitt nach der Linie II-II der Fig. 1 und
Fig. 3 eine Ausführungsvariante einer erfindungsgemäßen Vorrichtung zur Schnarchverhinderung in einem schematischen Blockschaltbild.

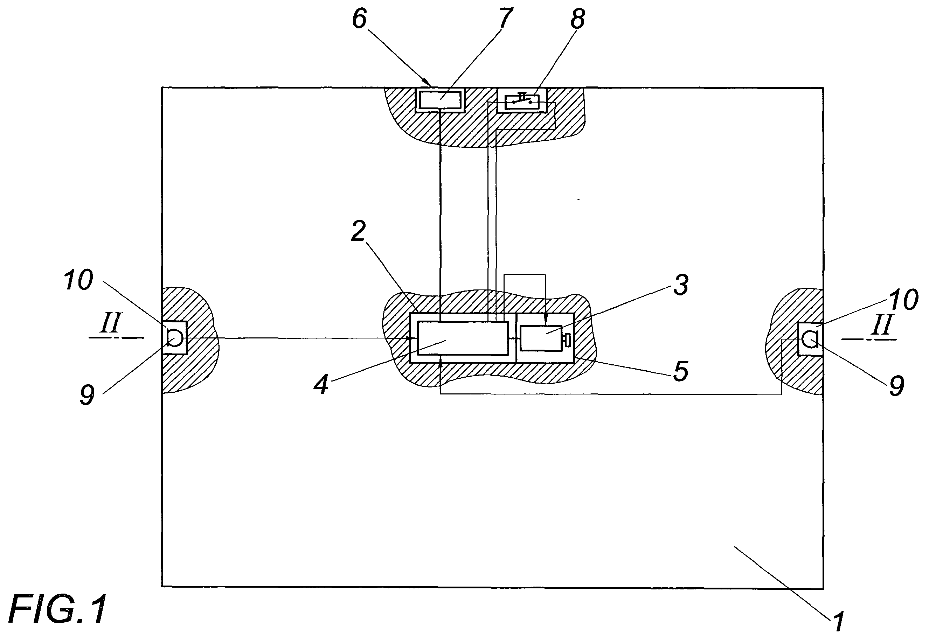

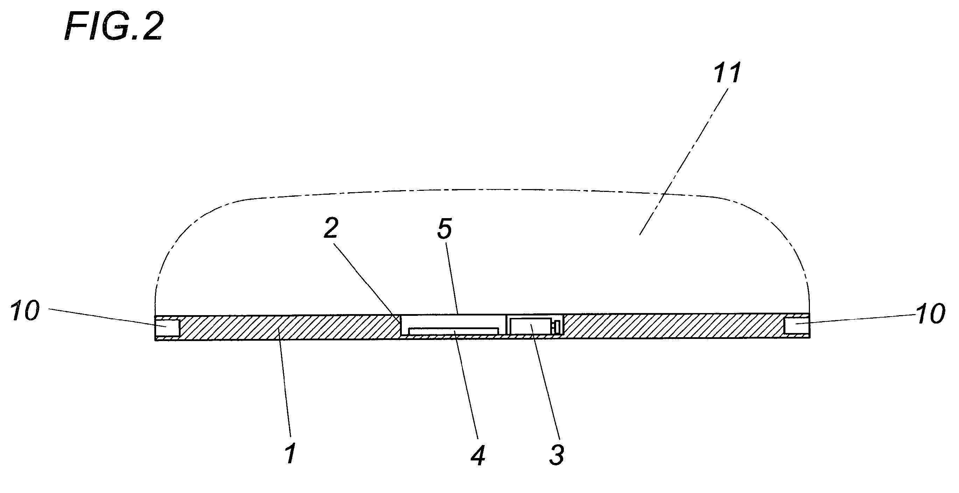

Die Vorrichtung zur Schnarchverhinderung nach den Fig. 1 und 2 weist eine Schwingungsübertragungsplatte 1 mit einer mittigen Ausnehmung 2 auf, in der einerseits ein elektrischer Unwuchtmotor 3 und anderseits eine Steuereinrichtung 4 angeordnet sind. Um einfache Montageverhältnisse unter Wahrung vorteilhafter Bedingungen zur Schwingungsübertragung zu erhalten, können der Unwuchtmotor 3 und die elektrische Schaltung der Steuereinrichtung 4 in einem Gehäuse 5 untergebracht werden, das formschlüssig in die Ausnehmung 2 der Schwingungsübertragungsplatte 1 eingreift.

Zur Stromversorgung ist die Schwingungsübertragungsplatte 1 mit einem von einer Längsseite her zugänglichen Batteriefach 6 versehen, in das Batterien 7 austauschbar eingesetzt sind. Außerdem ist ein mit der Steuereinrichtung 4 verbundener Wahlschalter 8 in der Schwingungsübertragungsplatte 1 eingelassen, um die Drehzahl des Unwuchtmotors 3 stufenweise einstellen zu können. Die Erfassung von Schnarchgeräuschen erfolgt über zwei Mikrofone 9, die in Aussparungen 10 im Bereich von einander gegenüberliegenden Seitenrändern der Schwingungsübertragungsplatte 1 eingesetzt und an die Steuereinrichtung 4 angeschlossen sind. Wird demnach über eines der beiden Mikrofone 9 ein Schnarchgeräusch erfaßt, dessen elektrisches Signal einen vorgegebenen, vorzugsweise einstellbaren Schwellwert übersteigt, so wird über die Steuereinrichtung 4 der Unwuchtmotor 3 eingeschaltet, und zwar über ein Zeitglied, so daß die Schwingungsübertragungsplatte 1 durch den Unwuchtmotor 3 für eine entsprechende Zeitspanne in Schwingungen versetzt wird, die sich über einen Kopfpolster 11 auf den Kopf des Schlafenden übertragen und den Schlafenden veranlassen, mit dem Schnarchen aufzuhören, ohne aufzuwachen. Der Kopfpolster bildet gemäß der Fig. 2 zusammen mit der Schwingungsübertragungsplatte 1 eine Handhabungseinheit, so daß eine Verlagerung zwischen dem Kopfpolster 11 und der Schwingungsübertragungsplatte 1 ausgeschlossen werden kann. Der Kopfpolster 11 kann in diesem Fall vorteilhaft aus einer Kopfauflage aus Naturlatex bestehen. Obwohl in unbelasteten Bereichen des Kopfpolsters 11 die Schwingungen der Schwingungsübertragungsplatte 1 nur weitgehend gedämpft übertragen werden können, ergibt sich im Kopfbereich eine ausreichende Schwingungsübertragung aufgrund der durch das Gewicht des Kopfes bedingten Verdichtung des Kopfpolsters 11.

Da im gesamten Bereich der Kopfauflage des Kopfpolsters 11 diese Schwingungsübertragung möglich ist, wird eine weitgehende Unabhängigkeit von der jeweiligen Kopflage und damit von der Schlafstellung des Schlafenden erreicht. Es muß ja davon ausgegangen werden, daß üblicherweise der Kopf in jeder Schlafstellung im Bereich des Kopfpolsters 11 zu liegen kommt. Damit ist aber eine freie, unbehinderte Bewegung des Schlafenden gewährleistet, der die Vorrichtung zur Schnarchverhinderung nicht wahrnimmt.

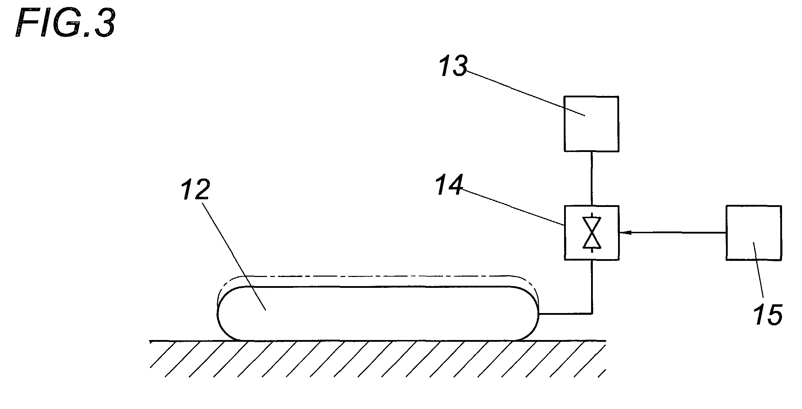

Eine andere Möglichkeit der Ausbildung einer Stelleinrichtung zur Schwingungs- bzw. Impulsübertragung auf den Kopf eines Schlafenden ist in der Fig. 3 dargestellt. Die Stelleinrichtung besteht aus einem aufblasbaren Kissen 12, vorzugsweise aus einem gummielastischen Werkstoff, das selbst als Kopfauflage oder als Unterlage für einen Kopfpolster dienen kann. Das Kissen 12 ist an eine Druckmittelquelle 13 über eine Ventilschaltung 14 angeschlossen, die über eine Steuereinrichtung 15 betätigt wird, und zwar in Abhängigkeit von auftretenden Schnarchgeräuschen, die über Mikrofone erfaßt werden, wie dies im Zusammenhang mit den Fig. 1 und 2 näher ausgeführt wurde. Wird die Ventilschaltung 14 über die Steuereinrichtung 15 angesteuert, so wird beispielsweise die Druckluftverbindung zwischen der Druckmittelquelle 13 und dem Kissen 12 kurzzeitig geöffnet, was mit einer impulsartigen Druckmittelbeaufschlagung des Kissens und in der Folge davon mit einer impulsartigen Kopfbewegung eines Schlafenden verbunden ist. Nach der Impulsbeaufschlagung wird über die Ventilschaltung 14 Druckmittel aus dem Kissen 12 abgelassen, bis der ursprüngliche Füllzustand wieder erreicht ist, was über eine Druckbegrenzung durch die Ventilschaltung 14 in einfacher Weise sichergestellt werden kann. Die in dieser Art erzeugbaren Druckimpulse können einzeln oder in einer vorgegebenen Folge abgegeben werden, womit eine gute Anpassung an die jeweiligen Verhältnisse möglich ist, zumal auch die Amplitude der Druckimpulse einfach über die Ventilsteuerung 14 eingestellt werden kann.

## Patentansprüche

1. Vorrichtung zur Schnarchverhinderung mit einem über eine Steuereinrichtung (4, 15) ansteuerbaren Stelleinrichtung für eine Kopfauflage eines Schlafenden, **dadurch gekennzeichnet, daß** die Stelleinrichtung die Kopfauflage nach einer Schwingungs- oder Impulsanregung in die Ausgangslage zurückstellt.

2. Vorrichtung nach Anspruch 1, **dadurch gekennzeichnet, daß** die Stelleinrichtung aus einem mit einer Schwingungsübertragungsplatte (1) verbundenen, elektrischen Unwuchtmotor (3) besteht und daß die Schwingungsübertragungsplatte (1) eine Unterlage für einen Kopfpolster (11) bildet.

3. Vorrichtung nach Anspruch 2, **dadurch gekennzeichnet, daß** der Unwuchtmotor (3) in einer Ausnehmung (2) der Schwingungsübertragungsplatte (1) vorgesehen ist.

4. Vorrichtung nach Anspruch 3, **dadurch gekennzeichnet, daß** der Unwuchtmotor (3) und die Steuereinrichtung (4) in einem Gehäuse (5) angeordnet sind, das in die Ausnehmung (2) der Schwingungsübertragungsplatte (1) formschlüssig eingesetzt ist.

5. Vorrichtung nach einem der Ansprüche 2 bis 4, **dadurch gekennzeichnet, daß** die Drehzahl des Unwuchtmotors (3) über einen Wahlschalter (8) einstellbar ist.

6. Vorrichtung nach einem der Ansprüche 2 bis 5, **dadurch gekennzeichnet, daß** zwei an die Steuereinrichtung (4) angeschlossene Mikrofone (9) zur Ansteuerung des Unwuchtmotors (3) in Abhängigkeit von durch die Mikrofone (9) empfangenen Schnarchgeräuschen in je einer Aussparung (10) der Schwingungsübertragungsplatte (1) im Bereich von einander gegenüberliegenden Seitenrändern vorgesehen sind.

7. Vorrichtung nach einem der Ansprüche 2 bis 6, **dadurch gekennzeichnet, daß** die Schwingungsübertragungsplatte (1) mit dem eine nachgiebige Kopfauflage beispielsweise aus Naturlatex bildenden Kopfpolster (11) zu einer Handhabungseinheit verbunden ist.

8. Vorrichtung nach Anspruch 1, **dadurch gekennzeichnet, daß** die Stelleinrichtung aus einer über die Steuereinrichtung (15) an ein aufblasbares Kissen (12) anschließbaren Druckmittelquelle (13) besteht.
